# EUROPEAN PATENT APPLICATION

(11) **EP 2 027 857 A2**
(43) Date of publication of application: **25.02.2009**
(21) Application number: 08252766.4
(22) Date of filing: 20.08.2008
(51) Int. Cl.: A61K 31/455, A61K 33/00, A61K 31/417, A61K 31/5575, A61K 31/7076, A61K 38/04, A61K 31/21, A61K 31/34, A61K 31/04, A61K 31/522, A61K 31/519, A61K 31/4985, A61K 31/472, A61P 15/02, A61P 15/10, A61P 9/08

(54) **Anhydrous compositions useful for attaining enhanced sexual wellness**

(30) Priority: 21.08.2007 US 957087 P
(71) Applicant: Mcneil-PPC, Inc, Skillman, NJ 08558 (US)
(72) Inventor: Ahmad, Nawaz, Monmouth Junction, NJ 08852 (US); Joyce, Michael, Randolph, NJ 07869 (US); Pitt, Stephen, Franklin Park, NJ 08823 (US)
(74) Representative: Kirsch, Susan Edith

(57) **Abstract**

The invention relates to an anhydrous composition comprising a vasodilator and an acceptable carrier wherein the vasodilator is present in an amount effective to increase the blood flow when the composition is applied to human tissue. The compositions according to the invention are non-flushing.

## Description

This application claims the benefit of priority of U.S. Provisional Application Serial No. 60/957,087, filed August 21, 2007 and hereby incorporates by reference the subject matter set forth therein. Related applications include U.S. Patent Applications Serial Nos. 11/842487 and 11/842271, the disclosures of which are hereby incorporated by reference.

### BACKGROUND

An estimated forty percent of women experience sexual difficulties at some period during their life. Female sexual dysfunction includes complications with arousal, desire, orgasms and/or painful intercourse. Studies have shown that women only achieve an orgasm 25% of the time via sexual intercourse alone. In many cases the physiological factors can be attributed to decrease in blood flow to genital region, particularly to the clitoris.

Prescription and over-the counter medications, illicit drugs and alcohol abuse contribute to sexual dysfunction. There are separate lists of drugs or medications that cause disorder of desire, medications that cause disorder of arousal and medications that cause orgasmic dysfunction.

Estimates of the number of women who have sexual dysfunction range from 19 to 50% in "normal" outpatient populations and increase to 68 to 75% when sexual dysfunction or problems that are not dysfunctional in nature are included.

A decline in desire, arousal, and frequency of intercourse and an increase in dyspareunia or painful intercourse have also been associated with menopause.

However, there is also a large population of women who have sexual dissatisfaction that is not truly medically dysfunctional in nature or associated with menopause. This general population of women wishes to achieve sexual satisfaction or improve their sexual performance by achieving and/or enhancing the orgasm.

Products are currently on the market that claim to be invigorating lubricants or are intended to aid in stimulating the clitoris to increase the duration and intensity of climax. Most of these ingredients are claimed to be vasodilators that act to increase sensitivity. For example, niacin-containing products include Climatique, distributed by Climatique International, Inc., Ioxora, distributed by Ioxora Bio-Medical Co New York, New York 10175, Emerita Response, Manufactured by Emerita^{®}, Portland Oregon, OR 97205, and Vibrel^{®} manufactured for GlycoBiosciences, Inc, Campbellville, Canada. These niacin-containing products, however, are aqueous compositions and when applied to the skin, result in irritation, itching and/or redness of the skin also known as a "flushing" response, which lasts for considerably long duration.

Accordingly, there remains a need for women, and men, who wish to achieve or enhance sexual satisfaction or improve their sexual performance by achieving and/or enhancing the orgasm in a manner that is free from side effects. Also, there is a need for a test that can qualitatively and quantitatively determine the actual blood flow on the area of human skin and can also monitor changes in this blood flow. The methods and compositions of the present invention answer this need.

It has been discovered that anhydrous compositions comprising a vasodilator result in an increase in blood flow but do not cause flushing or redness of the skin. Specifically, the vasodilatation caused by the compositions of this invention is controlled because the anhydrous base is responsible for penetration of the niacin and/or other effective vasodilators to the deeper layers of the tissue, which we theorize penetrates at least through the stratum corneum and preferably the epidermis. This results in a desired increase in blood flow without the undesired side effect of flushing.

### SUMMARY OF INVENTION

Accordingly, the invention relates to an anhydrous composition comprising a vasodilator and an acceptable carrier wherein the vasodilator is present in an amount effective to increase the blood flow when the composition is applied to human tissue. The anhydrous compositions according to the invention preferably contain less than 20% water, more preferably less than about 5% water and most preferably, less than about 3% water.

In another embodiment, the invention relates to a method of attaining enhanced sexual response or sexual wellness of an individual comprising administering to the genital areas of the individual, an anhydrous composition comprising an effective amount of a vasodilator.

In yet another embodiment, the invention relates to a method for measuring the efficacy of a composition for improving sexual wellness comprising:
(a) establishing a baseline sexual wellness value by measuring the blood flow on a target area of an individual;
(b) after step (a), administering said composition to the target area;
(c) after step (b), measuring a blood flow value on the target area;
(d) comparing the value obtained in step (a) with the value obtained in step (c) wherein the difference between the value obtained in step (c) and the value obtained in step (a) signifies the magnitude of the increase or decrease in the sexual wellness of said individual.

### BRIEF DESCRIPTION OF THE FIGURES

A more particular description of the invention, briefly summarized above may be had by reference to the embodiments thereof that are illustrated in the appended figures. It is to be so noted, however, that the appended figures illustrate only typical embodiments of the invention and, therefore, are not to be considered limiting of its scope, for the invention may admit to other equally effective embodiments.
Figure 1 is a bar graph depicting the blood flow flux monitored by Laser Doppler Imaging ("LDI") of the skin of a subject's arm immediately after and three minutes after the application of the compositions of Examples 2 and 3. Three (3) ml of each composition was manually rubbed onto the forearm of the subject.
Figure 2 is a bar graph depicting the percent blood flow changes from baseline monitored by LDI after 3 minutes of application. Three (3) ml of each composition for Examples 2 (left arm) and 3 (right arm)) was manually rubbed onto the forearm of the subject for three (3) minutes.
Figure 3 is an LDI image of the skin of the right and left arms after application for 3 minutes of the compositions of Example 2 (left arm) and Example 3 (right arm). Red shows the highest blood flow and blue shows areas of lower % blood flow change. Figure 3 is the photograph image of Figure 2.
Figure 4 is a bar graph of the blood flow changes from baseline monitored by LDI after 2 ml of the compositions of Examples 11-15 were manually rubbed for three (3) minutes on the forearm of the subject in a separate test for each Example at a different time.
Figure 5 is a bar graph of the blood flow changes from baseline monitored by LDI after 3ml of the compositions of Examples 16-20 were manually rubbed onto the forearm of a subject for 3 minutes in a separate test at a different time for each Example. Compositions of Example 19 and Example 20 were compared with the Placebo (Example 18) separately when the LDI test was run for Examples 19 and 20.
Figure 6 is a bar graph comparing the blood flow changes from baseline monitored by LDI when 3 ml of each of Examples 21 and 28 were manually rubbed on the left forearm and right forearm of the subject respectively for 3 minutes in the same LDI test.
Figure 7 is an LDI image of the skin of the right and left arms after application as described for Figure 6 for 3 minutes of the compositions of Example 21 (left arm) and Example 28 (right arm).
Figure 8 is a bar graph comparing the blood flow changes from baseline monitored by LDI when 3 ml of each of Examples 3 and 29 were manually rubbed on the left forearm and right forearm of the subject respectively for 3 minutes in the same LDI test.
Figure 9 is the LDI picture of Figure 8 showing higher % increase in blood flow as represented by greater red and blue area covered for Example 3 as compared with Example 29 (Zestra) showing lower % increase in blood flow as shown by smaller red and blue covered area.
Figure 10 is a bar graph comparing the blood flow changes from baseline monitored by LDI when 3 ml of each of Examples 4 and 1 were manually rubbed on the left forearm and right forearm of the subject respectively for 3 minutes in the same LDI test. LDI test was run for 60 minutes and LDI readings of % blood flow change were recorded after 3 minutes (immediately after treatment), 15 minutes, 35 minutes and 55 minutes intervals.
Figure 11 is the LDI picture of Figure 10 showing progressive decrease in % blood Flow for both Example 1 and Example 4.
Figures 12 and 13 are bar graphs comparing the blood flow changes from baseline monitored by LDI when 0.3 ml of each of Examples 30A, 30B, 31A, 31B, 32A and 32B were manually rubbed for 30 seconds on the left and right forearm of the subject in a separate test for each of the Examples A & B pair. The LDI readings of % blood flow change in Figures 12 and 13 were recorded 5 minutes and 30 minutes after treatment, respectively. Formulations A were applied to the left arm and formulations B were applied to the right arm of the subject.
Figure 14 is a bar graph comparing the percent change in blood flow monitored by LDI when 0.3 ml of each of Examples 22, Example 30B, Example 31B and Example 32B were manually rubbed on the right forearms of the subject in separate tests for 30 seconds. The LDI readings of % blood flow change were recorded 5 and 30 minutes after treatment. Figure 14 illustrates the percent change in blood flow of Examples 30B, 31B and 32B as compared with that of Example 22.

### DETAILED DESCRIPTION OF THE INVENTION

It is believed that one skilled in the art can, based upon the description herein, utilize the present invention to its fullest extent. The following specific embodiments are to be construed as merely illustrative, and not limitative of the remainder of the disclosure in any way whatsoever.

Unless defined otherwise, all technical and scientific terms used herein have the same meaning as commonly understood by one of ordinary skill in the art to which the invention belongs. Any percentage (%) concentration of a component is weight by weight (w/w) unless otherwise indicated.

This invention relates to sexual enhancement compositions for use by both the male and the female. These sexual enhancement compositions work by increasing the blood flow to the sexual areas of both the male and female. Since the target area of these compositions is local, these compositions do not cause side effects from systemically-administered erectile dysfunction medications such as VIAGRA^{®} or other medications that are similar in mechanism in the males and undesirable side effects of other active ingredients in the compositions used for FSD (Female Sexual Dysfunction), such as topically-administered testosterone or other hormone-containing medications that are topically or systemically administered. Such undesirable side effects include, for example, decrease in blood pressure, formation of blood clots, heart attacks and cancer.

The main objects of the sexual enhancement compositions of this invention are as follows:
- Vasodilation to increase the blood flow in the clitoris and vagina.
- To increase the sensitivity or provide enhanced sexual sensation.
- Avoiding flushing.

These objects are accomplished through the administration of the anhydrous compositions of the invention comprising, consisting essentially of, and consisting of a vasodilator and an acceptable carrier. Suitable vasodilators include niacin derivatives such as nicotinic acid also called Niacin or Vitamin B3, nicotinates, such as, methyl nicotinate, benzyl nicotinate, nicotinamide, methyl niconate and niacinamide. We believe that other vasodilators, in particular, vasodilators capable of penetration to the deeper layers of the tissue at least through the stratum corneum and preferably the epidermis but do not cause flushing would be useful to achieve the desired results in the compositions and methods of this invention. Vasodilators may be categorized as being endogenous (i.e., normally produced within the body) or exogenous (i.e., normally produced outside of the body). Endogenous vasodilators preferably include nitric oxide, histamine, prostaglandin D2, adenosine, L-arginine, bradykinin as well as niacin and the like. Exogenous vasodilators include nitric oxide inducers and PDE5 inhibitors. Nitric oxide inducers include glyceryl nitrate (commonly known as nitroglycerin), isosorbide mononitrate, isosorbide dinitrate, pentaerythrotol tetranitrate and sodium nitroprusside. PDE5 Inhibitors include sildenafil, tadalafil, vardenafil, theobromine and papaverine and the like.

In one embodiment, a preferred vasodilator is one that normally causes flushing in aqueous compositions but does not cause flushing in the compositions according to the invention. In yet another embodiment, the compositions according to the invention do not contain a niacin derivative or contain, for example less than 0.1%, of a niacin derivative.

In one embodiment nicotinic acid or niacin is the preferred derivative as methyl nicotinate has been found to have a strong undesirable odor. Generally, the niacin derivative is present in the anhydrous composition in an amount effective to increase the blood flow to human tissue. In one embodiment the vasodilator is present in an amount ranging from about 0.1 to about 0.5% by weight, for example from about 0.1 to about 0.2% by weight. Depending upon their potency, other exogenous and endogenous vasodilator ingredients may be present in the compositions of this invention in smaller ranges, i.e., preferably from about 0.05 to about 0.15% by weight, more preferably from about 0.08 to about 0.12% by weight and most preferably, about 0.1% by weight.

The anhydrous compositions of the invention comprise an acceptable carrier. By "acceptable carrier" it is meant any non-aqueous carrier that will not interfere with the object of this invention. Suitable acceptable carriers include polyhydric alcohols as described in copending U.S. Patent Application Serial No. 11/403,592, filed April 13, 2006, the disclosure of which is hereby incorporated by reference. Examples include polyethylene glycol (hereinafter, "PEG"). Polyethylene glycol ethers may also be used, including PEG ethers of propylene glycol, propylene glycol stearate, propylene glycol oleate and propylene glycol cocoate and the like. Specific examples of such PEG ethers include PEG-25 propylene glycol stearate, PEG-55 propylene glycol oleate and the like. Preferably, at least one of the polyhydric alcohols of the compositions of this invention is a polyalkylene glycol or others selected from the following group: glycerine, propylene glycol, butylene glycol, hexalene glycol or polyethylene glycol of various molecular weight and the like and/or combination thereof. More preferably, the compositions of this invention contain a polyethylene glycol; most preferably, the polyethylene glycol may be selected from the following group: polyethylene glycol 400 or polyethylene glycol 300. Polypropylene glycol of various molecular weights may also be used. PEGylated compounds such as peptide or protein derivatives obtained through PEGylation reactions may also be used. In addition, block copolymers of PEG's may be used, such as (ethylene glycol)-block-poly(propylene glycol)-block-(polyethylene glycol), poly(ethylene glycol-ran-propylene glycol) and the like. The compositions of this invention should contain polyhydric alcohols in an amount from about 80% to about 98% by weight of the composition.

Preferably, the compositions of this invention contain at least one polyhydric alcohol, and more preferably, at least two polyhydric alcohols. Preferably the polyhydric alcohol portion of the compositions of this invention may include one or more polyhydric alcohols such as alkylene glycols and others selected from the following group: glycerin, propylene glycol, butylene glycol, hexalene glycol or polyethylene glycol of various molecular weight and the like and/or combination thereof. More preferably, the compositions of this invention contain a polyethylene glycol; most preferably, the polyethylene glycol may be selected from the following group: polyethylene glycol 400 or polyethylene glycol 300. The compositions of this invention should contain polyhydric alcohols in an amount from about 80% to about 98% by weight of the composition.

In a preferred embodiment, the carrier is a mixture of polyethylene glycol and propylene glycol as described in U.S. Patent No. 7,005,408, the disclosure of which is hereby incorporated by reference. For example, the polyhydric alcohol is a mixture of polyethylene glycol, for example polyethylene glycol 400, and propylene glycol wherein the weight ratio of polyethylene glycol to propylene glycol is about 3:1.

It has been observed that polyethylene glycols in an anhydrous form degrade much more readily as compared to their aqueous solutions. This degradation of polyethylene glycols can result in the development of a formaldehyde type of odor. Antioxidants may be included to prevent the development of this odor. Examples of suitable antioxidants include α-tochopherol, α-tochopherol acetate, butylated hydroxytoluene (BHT), ascorbic acid, tocopherol and propyl gallate and mixtures thereof and the like as described in copending U.S. Patent Application Serial No. 11/403,592, filed April 13, 2006, the disclosure of which is hereby incorporated by reference. The antioxidant may be present for example, in amounts ranging from about 0.05% to about 3% by weight, preferably from 0.05% to about 1.5% by weight.

In one embodiment, the compositions according to the invention may include a sensory agent that provides a cue to the user that vasodilation and/or engorgement that leads to arousal is taking place as described for example in copending U.S. Provisional Patent Application, Serial No. 60/889,062, Patent Applications Serial Nos. 11/842487 and 11/842271, the disclosures of which are hereby incorporated by reference. Examples of sensory agents include methyl salicylate, menthyl lactate and methyl nicotinate.

In one embodiment, the compositions of the invention further comprise at least one sensitivity enhancer to enhance sensitivity. Generally, the sensitivity enhancer may be present in amounts ranging from about 0.05 to about 5% by weight. Although their primary role is sensitivity enhancement, these fall into two separate categories.

The first category of these sensitivity enhancers are cooling compounds, especially non-menthol cooling compounds, such as, described, for example, in Cool Without Menthol & Cooler Than Menthol by John C. Lefingwell, Ph.D., Leffingwell & Associates, April 19, 2007. These include WS-23 (2-Isopropyl-N, 2,3-trimethylbutyramide), WS-3 (N-Ethyl-p-menthane-3-carboxamide) and WS-5 [Ethyl 3-(p-menthane-3-carboxamido) acetate] supplied by Millennium Specialty Chemicals, 601 Crestwood Street, Jacksonville, Fl 32208-4476, USA. Of these, WS-5 is the "coldest" of commercially available "coolants" and has recently received GRAS approval. Also included is Menthone glycerol ketal (sold as Frescolat^{®} MGA by Haarmann & Reimer). Both the racemic and leavo-forms appear on the FEMA GRAS list but the leavo-form appears to be the item of commerce. (-)-Menthyl lactate (sold as Frescolat^{®} ML by Haarmann & Reimer. Also included is (-)-Isopulegol sold under the name "Coolact P^{®} " by Takasago International.

An example of a second category of sensitivity enhancers are warming compounds that work either by exothermic reaction or by activation of chemoreceptors for heat. These include piperine from Piper nigrum or Black and White Pepper, 1- Acetoxychavicol Acetate, a pungent principal from Alpina Galangal, Shansools, specifically, α - hydroxyshansool from Sichuan pepper and Ginger Extract available from Givaudan Fragrances Corporation, 1775 Windsor Road, Teaneck, New Jersey 07666, USA and Timurol, from Napalese pepper, available from Monell Chemical senses Center, 3500 Market Street, Philadelphia, PA 19104-3308. These compounds give interesting warming and tingling sensation. Also included in this category is Hesperidin and specifically glucosyl hesperidin supplied by Hayashibara International, Fetcham park House, Lower Rod, Fetcham, Leatherhead, Surrey, KT229HD, U.K

The third category of sensitivity enhancement is tingling compounds that are different from cooling or warming compounds. These compound compounds cause or generate a feeling of buzz or vibration, which is pleasant. These include the following: Shansools, specifically α - hydroxyshansool from Sichuan pepper, distributed by Jivaudan SA, 5, Chemin de la Parfumerie CH-1214 Vernier, Genève, Switzerland and Spilanthol derived by Jumboo Extract, distributed by Takasago International Group, 4 Volvo drive, P.O.Box 932, Rockleigh, NJ 07647 -0923. These also include Timurol, from Nepalese pepper by Monell Chemical Senses Center, 3500 Market Street Philadelphia, PA 19104-3308

Compositions of this invention also include cellulose based lubricating and viscosity agents as described in U.S. Patent No. 7,005,408, the disclosure of which is incorporated by reference. Examples include carboxymethylcelluloe, hydroxyetylcellulose, hydroxypropyl methylcellulose, especially hydroxypropylcellulose sold under the name Klucel HF, distributed by Aqualon Inc, Delaware. Such cellulose based lubricating and viscosity agents may be incorporated at about 0.05% to about 5% by weight, for example, 0.4% to about 3% by weight.

In one embodiment, the compositions according to the invention contain from about 0.10% to about 0.2% by weight of vasodilator, e.g., niacin derivative, preferably niacin, from about 0.05% to about 1.5% by weight of sensitivity enhancement agent or combination thereof, from about 20% to about 80% polyethylene glycol 400, from about 20% to about 80% of propylene glycol, from about 0.2% to about 1.5% hydroxypropylcellulose and from about 0.05 to about 1.5% or from about 0.05% to about 3%, α-tochopherol, or 0.05 to 1.5% or from about 0.05% to about 3%, α-tochopherol acetate.

The compositions of this invention can be prepared using techniques known in the art for preparing anhydrous compositions. See for example, U.S. Patent No. 7,005,408, the disclosure of which is hereby incorporated by reference. For example, an acceptable carrier, e.g., propylene glycol and/or polyethylene glycol 400, and optionally a lubricating and viscosity agent, e.g., Klucel HF are mixed at about 50°C (45°C -55°C) until a uniform gel is obtained.

Into the above gel, the vasodilator is added with constant mixing until completely dissolved. Wherever applicable, sensitivity enhancers and other optional ingredients can also be added.

The batch is cooled to room temperature with continued mixing. If desired, antioxidants are and mixed until these completely dissolved.

The compositions of this invention may be applied to the human tissue, for example, the genital region of a male or female, the skin or mucous membranes, preferable the vaginal or oral mucosa as described in U.S. Patent No. 7,005,408, the disclosure of which is hereby incorporated by reference. The compositions of this invention may be a liquid, a semi-solid, or a solid depending upon the particular intended use thereof. The compositions of this invention may also be formulated into soft or hard gelatin capsules, suppositories and impregnated into fabrics or polymers. Compositions of this invention may be manufactured as a coating of a tampon, or dispersing throughout the absorbent tampon material, or enclosed inside as a core of a tampon.

In one embodiment of the invention, the compositions of the invention are administered between about 5 to about 30 minutes prior to intercourse. Further, it is desired that blood flow in the areas that were treated is restored to the normal blood flow within a short time period, for example, within one hour, preferably, less than an hour after intercourse.

The compositions and methods of this invention unexpectedly result in an increase in the blood flow but do not cause "flushing" of the skin. As used herein, "flushing" is a skin reaction to a topically-applied material which includes the symptoms of redness, swelling, itching and/or irritation at the site of application. Overall interaction of the anhydrous carrier along with the vasodilatation provided by the active vasodilators results in an effective and desired increase in blood flow. The preferred vasodilator used by this invention is niacin or nicotinic acid. As discussed above, in niacin containing aqueous compositions, for example, Vibrel, manufactured by GlycoBiosciences Inc., Campbellville, Canada, there is prolonged "flushing" and redness of the skin and tissues. This is due to the fact that niacin in the composition stays in the exterior layers of the skin.

In the compositions according to the invention, the vasodilatation is controlled because of the amount of vasodilator, such as, niacin derivative, preferably niacin, used (0.1% to 0.5%) and because the unique anhydrous base is responsible for penetration of the vasodilator to the deeper layers of the tissue, which we theorize penetrates at least through the stratum corneum and preferably the epidermis. This results in a desired increase in blood flow without an undesired flushing effect.

In another embodiment the invention relates to the use of Laser Doppler Imaging to measure the blood flow and an increase in the blood flow in the skin. Accordingly, the invention also relates to a method for measuring the efficacy of a composition for improving sexual wellness comprising:
(a) establishing a baseline sexual wellness value by measuring the blood flow on a target area of an individual;
(b) after step (a), administering said composition to the target area;
(c) after step (b), measuring a blood flow value on the target area;
(d) comparing the value obtained in step (a) with the value obtained in step (c) wherein the difference between the value obtained in step (c) and the value obtained in step (a) signifies the magnitude of the increase or decrease in the sexual wellness of said individual.

Laser Doppler Imaging ("LDI") is a technique commonly used to monitor blood flow in the skin. LDI analysis utilizes low power laser light to penetrate the skin (less than about 0.2 mm) and interact with moving blood cells. A photodetector is used to measure the frequency of the backscattered light. Due to the Doppler effect, moving blood cells will cause a frequency change in the backscattered light whereas non-moving tissue will scatter light back at the same frequency. The frequency change is directly proportional to the number of moving cells (blood flow). Using this principle, LDI is used to scan skin areas and results in a two-dimensional skin perfusion image of the skin.

Overall female sexual wellness, or female sexual well-being, may also be measured utilizing a Female Sexual Well-Being (FSWB) Scale. There are five main domains of FSWB that can be measured, including: (1) interpersonal sex, (2) emotional sex, (3) arousal-natural lubrication, (4) orgasm-satisfaction, and (5) artifical lubrication. These domains address four major aspects of Female Sexual Well-Being: (1) physical response and sensation, (2) emotional and cognitive components, (3) contextual factors and (4) communication. It is expected that using the compositions and methods of this invention will improve the FSWB scores of individuals initially scoring low in the five main domains of FSWB and/or maintain FSWB scores of those scoring high in these domains after using the compositions and methods of this invention.

LDI Test Procedure used by this invention utilizes Moor Instruments, MoorLDI2-IR to measure the blood flow in the forearm before and after the application of various test samples. Alternately, the Periscan PIM High Resolution Laser Doppler Imager (HR) by Perimed AB, Box 564, SE-17526 Jarfalla, Stockholm, Sweden can be also used. A suitable amount, for example, from 1 ml to 3 ml of the Test Sample is applied to the forearm and rubbed into the skin lightly for 3 minutes. Compositions of this invention containing higher potency vasodilators may be applied in suitable amount from about 0.1 to about 0.5 ml, more preferably from about 0.2 to about 0.4 ml and most preferably about 0.3 ml.

Samples of compositions to be tested may be applied as follows: Area of forearms between elbow to the upper portion of the hand is washed with soap and water and dried using a paper towel. After waiting for approximately 10 minutes a sample of the composition to be tested is filled to a 3 ml level in a 5 ml plastic syringe. The contents of the syringe may now be carefully expressed over the middle of one of the forearms. Using the index and the middle finger of the other hand the sample is evenly spread over the entire forearm and is gently rubbed over the entire arm for a duration of about 3 minutes. Now the same procedure is repeated for the reference baseline sample over the other arm.

Both arms may now be placed on the platform under the laser beam of the LDI equipment and scanned for a period of 3 minutes.

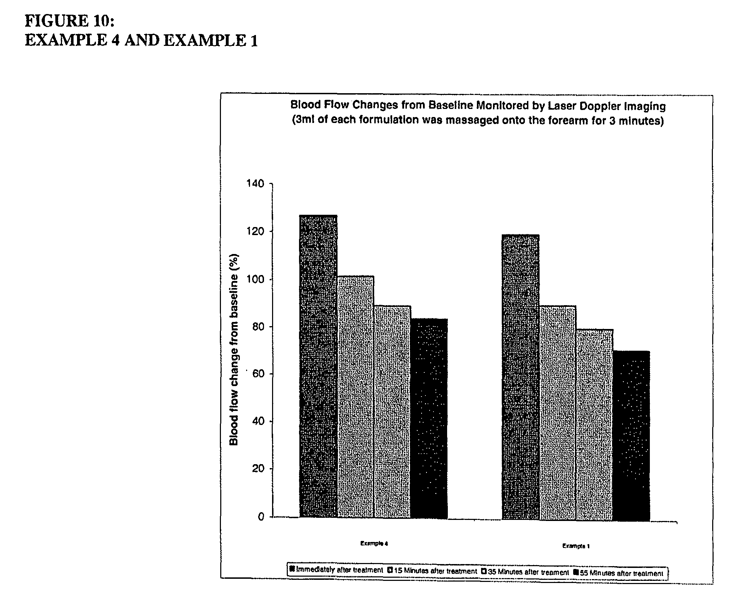

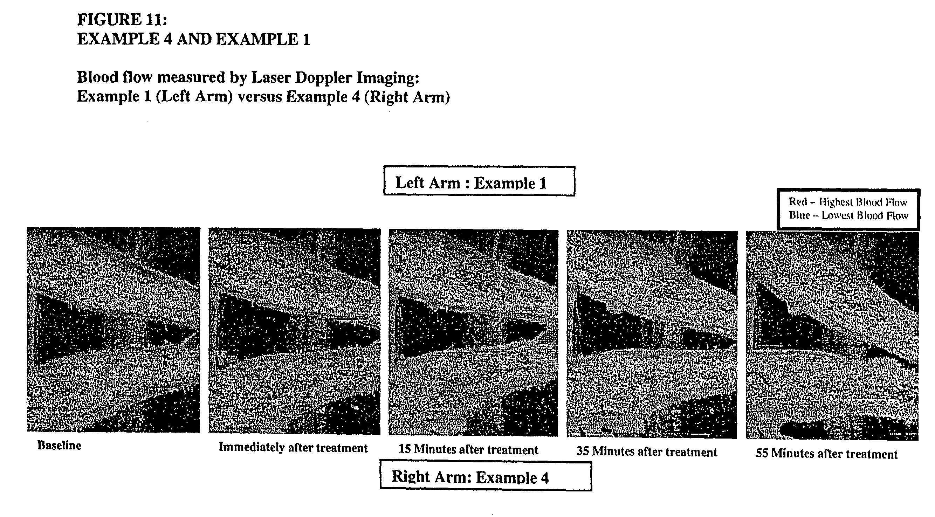

LDI scanning is normally conducted before sample application as well as 3 minutes after the application of the sample. Because the increase in blood flow results in the engorgement of the tissues, especially the vaginal area, it is most desirable that after the sexual activity, the blood flow is restored to the normal blood flow. To ascertain that normal blood flow is restored, in a special experiment LDI observations of the blood flow were conducted at 0, 15, 35 and 55 minutes after application of the sample, as reported in Figures 10 and 11 in which samples of compositions of Example 1 and Example 4 were used. The results of this experiment confirmed that the blood flow has a gradual decreasing trend ranging for a decrease of 36% for Example 4 to about 50% for Example 1 in a duration of 55 minutes.

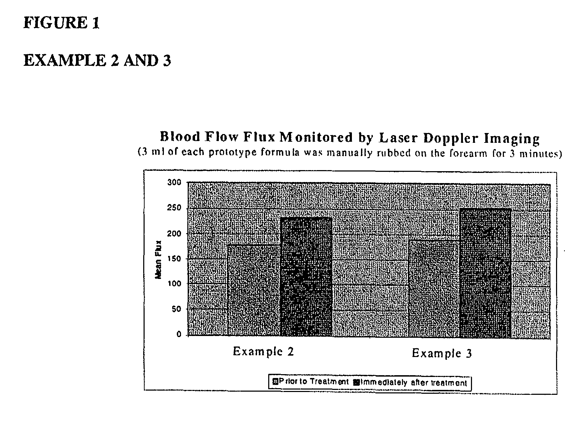

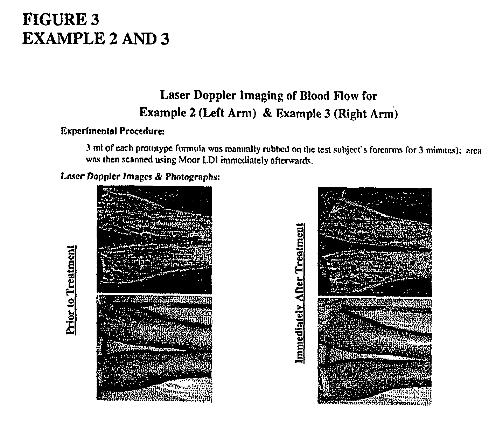

Blood flow values are calculated using the LDI Moor image analysis software and average blood flow values (in arbitrary units) is calculated at each time point. A bar graph showing the quantitative blood flow increase after application of formulation is shown, for example, in Figures 1,2,4 and 5, 6, 8, and 10 and LDI images are shown in Figures 3, 7, 9, and 11.

As discussed above, the compositions according to the invention are non-flushing. Generally, an increase in blood flow that is greater than 300% will cause flushing. Accordingly, in one embodiment, the compositions according to the invention demonstrate an increase in blood flow that is less than 300%, preferably from about 50% to about 150%.

The invention will now be illustrated by means of the nonlimiting examples that follow.

### EXAMPLES

### Example 1.

| Ingredient | (% w/w) |
|---|---|
| Polyethylene Glycol 400 | 75.00 |
| Propylene Glycol | 24.60 |
| Hydroxypropylcellulose (Klucel HF) | 0.30 |
| Dl-A-Tocopherol (Vitamin E Alcohol) | 0.10 |
| | |
| Total | 100.00 |

The composition of Example 1 was made as follows:
1. Into the manufacturing container the following were added
   Propylene Glycol
   Polyethylene Glycol 400
   Klucel HF
2. Mixed using a Silverson Mixer while heated to about 50°C (45°C -55°C) until a uniform gel was obtained.
3. Cooled to room temperature
4. Added α - Tocopherol and mixed until dissolved.

### Example 2.

| Ingredient | (% w/w) |
|---|---|
| Niacin (Nicotinic Acid) | 0.10 |
| Polyethylene Glycol 400 | 75.00 |
| Propylene Glycol | 24.50 |
| Hydroxypropylcellulose (Klucel HF) | 0.30 |
| Dl-A-Tocopherol (Vitamin E Alcohol) | 0.10 |
| | |
| Total | 100.00 |

The composition of Example 2 was made as follows:
1. Into the manufacturing container the following were added:
   Propylene Glycol
   Polyethylene Glycol 400
   Klucel HF
   Niacin
2. Mixed using a Silverson Mixer while heated to about 50°C (45°C -55°C) until a uniform gel was obtained.
3. Cooled to room temperature
4. Added α - Tocopherol and mixed until dissolved.

### Example 3.

| Ingredient | (% w/w) |
|---|---|
| Niacin (Nicotinic Acid) | 0.50 |
| Polyethylene Glycol 400 | 75.00 |
| Propylene Glycol | 24.10 |
| Hydroxypropylcellulose (Klucel HF) | 0.30 |
| Dl-A-Tocopherol (Vitamin E Alcohol) | 0.10 |
| | |
| Total | 100.00 |

The composition of Example 3 was made as follows:
1. Into the manufacturing container the following were added
   Propylene Glycol
   Polyethylene Glycol 400
   Klucel HF
   Niacin
2. Mixed using a Silverson Mixer while heated to about 50°C (45°C -55°C) until a uniform gel was obtained.
3. Cooled to room temperature
4. Added α-Tocopherol and mixed until dissolved.

As demonstrated by Figure 1, the mean blood flow flux was greater for Example 3, which contained 0.5% niacin, as compared to Example 2 containing 0.1% niacin. Flux is the "rate of flow across the area" or it is "the quantity of movement." Figure 1 shows the Flux or rate of flow side by side prior to the application of the sample and 3 minutes after application of the sample.

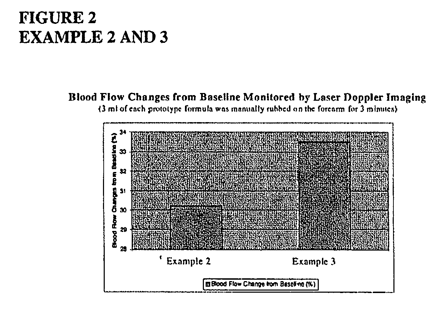

Figure 2 further demonstrates that the percent blood flow change from baseline is greater for Example 3 containing 0.5% niacin as compared with Example 2 containing 0.1% niacin. Figure 2 shows the difference between the rate of flow prior to the treatment and after the treatment, calculated on % basis. For example in Figure 1 for Example 3 (right graph) the Flux prior to treatment is approximately 190 and after the treatment it is approximately 255. The difference is 65. Dividing 65 by 190 and multiplying the result by 100, we arrive at approximately 34%, which is very close to Example 3 in Figure 3.

As demonstrated by Figures 1 and 2, the greater percentage of niacin in the composition the great the increase in blood flow.

Figure 3 depicts the Laser Doppler Imaging ("LDI") image of the skin of the right and left arms after application of the compositions of Example 2 (left arm) and Example 3 (right arm). The image of the left arm which was treated with the composition of Example 2 containing 0.1% Niacin indicated lower % blood flow change in comparison to the image of the right arm which was treated with the composition of Example 3 containing 0.5% Niacin showing higher % blood flow change. Red indicates the highest blood flow and blue indicates areas of lower % blood flow change.

### Example 4.

| Ingredient | (% w/w) |
|---|---|
| Niacin (Nicotinic Acid) | 0.30 |
| Nicotinamide | 0.20 |
| Polyethylene Glycol 400 | 75.00 |
| Propylene Glycol | 24.10 |
| Hydroxypropylcellulose (Klucel HF) | 0.30 |
| Dl-A-Tocopherol (Vitamin E Alcohol) | 0.10 |
| | |
| Total | 100.00 |

The above composition of Example 4 was prepared as follows:
1. Into the manufacturing container the following were added
   Propylene Glycol
   Polyethylene Glycol 400
   Klucel HF
2. Mixed using a Silverson Mixer while heated to about 50°C (45°C -55°C) until a uniform gel was obtained
3. Into the mixture in Step 2 the following were added with mixing until completely dissolved:
   Niacin
   Niacinamide
4. Cooled the mixture to room temperature
5.Added α - Tocopherol (Vitamin E alcohol) and mixed until dissolved.

Figure 10 shows that Blood Flow progressively decreases with time pointing to the safety of the application. Figure 10 is a bar graph comparing the blood flow changes from baseline monitored by LDI when 3 ml of each of Examples 4 and 1 were manually rubbed on the left forearm and right forearm of the subject respectively for 3 minutes in the same LDI test. LDI test was run for 60 minutes and LDI readings of % blood flow change were recorded after 3 minutes (immediately after treatment), 15 minutes, 35 minutes and 55 minutes intervals. Figure 11 is the LDI picture of Figure 10 showing progressive decrease in % blood Flow for both Example 1 and Example 4.

### Example 5.

| Ingredient | (% w/w) |
|---|---|
| Niacin (Nicotinic Acid) | 0.10 |
| Ginger Extract | 0.10 |
| Polyethylene Glycol 400 | 75.00 |
| Propylene Glycol | 24.40 |
| Hydroxypropylcellulose (Klucel HF) | 0.30 |
| Dl-A-Tocopherol (Vitamin E Alcohol) | 0.10 |
| | |
| Total | 100.00 |

The above composition of Example 5 was prepared as follows:
1. In the manufacturing container the following were added:
   Propylene Glycol
   Polyethylene Glycol 400
   Klucel HF
2. Mixed using a Silverson Mixer while heated to about 50°C (45°C -55°C) until a uniform gel was obtained
3. Into the mixture in Step 2 the following were added with mixing until completely dissolved:
   Niacin
   Ginger Extract
4. Cooled the mixture to room temperature
5. Added α-Tocopherol (Vitamin E alcohol) and mixed until dissolved.

### Example 6.

| Ingredient | (% w/w) |
|---|---|
| Niacin (Nicotinic Acid) | 0.10 |
| Alpha Glucosyl Hisperidin | 0.10 |
| Polyethylene Glycol 400 | 75.00 |
| Propylene Glycol | 24.40 |
| Hydroxypropylcellulose (Klucel HF) | 0.30 |
| Dl-A-Tocopherol (Vitamin E Alcohol) | 0.10 |
| | |
| Total | 100.00 |

The above composition of Example 6 was prepared as follows:
1. In the manufacturing container the following were added
   Propylene Glycol
   Polyethylene Glycol 400
   Klucel HF
2. Mixed using a Silverson Mixer while heated to about 50°C (45°C -55°C) until a uniform gel was obtained
3. Into the mixture in Step 2 the following were added with mixing until completely dissolved:
   Niacin
   Alpha Glucosyul Hisperidin
4. Cooled the mixture to room temperature
5. Added α-Tocopherol (Vitamin E alcohol) and mixed until dissolved.

### Example 7.

| Ingredient | (% w/w) |
|---|---|
| Niacin (Nicotinic Acid) | .50 |
| Ginger Extract | 0.10 |
| Polyethylene Glycol 400 | 75.00 |
| Propylene Glycol | 24.00 |
| Hydroxypropylcellulose (Klucel HF) | 0.30 |
| Dl-A-Tocopherol (Vitamin E Alcohol) | 0.10 |
| | |
| Total | 100.00 |

The above composition of Example 7 was prepared as follows:
1. In the manufacturing container the following were added:
   Propylene Glycol
   Polyethylene Glycol 400
   Klucel HF
2. Mixed using a Silverson Mixer while heated to about 50°C (45°C -55°C) and mixed until a uniform gel was obtained
3. Into the mixture in Step 2 the following were added with mixing until completely dissolved:
   Niacin
   Ginger Extract
4. Cooled the mixture to room temperature
5. Added α-Tocopherol (Vitamin E alcohol) and mixed until dissolved.

### Example 8.

| Ingredient | (% w/w) |
|---|---|
| Niacin (Nicotinic Acid) | 0.50 |
| Alpha Glucosyl Hisperidin | 0.10 |
| Polyethylene Glycol 400 | 75.00 |
| Propylene Glycol | 24.00 |
| Hydroxypropylcellulose (Klucel HF) | 0.30 |
| Dl-A-Tocopherol (Vitamin E Alcohol) | 0.10 |
| | |
| Total | 100.00 |

The above composition of Example 8 was prepared as follows:
1. Into the manufacturing container the following was added:
   Propylene Glycol
   Polyethylene Glycol 400
   Klucel HF
2. Mixed Using a Silverson Mixer while heated to about 50°C (45°C -55°C) until a uniform gel was obtained
3. Into the mixture in Step 2 the following were added with mixing until completely dissolved:
   Niacin
   Alpha Glucosyl Hisperidin
4. Cooled the mixture to room temperature
5. Added α-Tocopherol (Vitamin E alcohol) and mixed until dissolved.

### Example 9.

| Ingredient | (% w/w) |
|---|---|
| Methyl Salicylate | 0.20 |
| Methyl Nicotinate | 0.20 |
| Polyethylene Glycol 400 | 75.00 |
| Propylene Glycol | 24.20 |
| Hydroxypropylcellulose (Klucel HF) | 0.30 |
| Dl-A-Tocopherol (Vitamin E Alcohol) | 0.10 |
| | |
| Total | 100.00 |

The above composition of Example 9 was prepared as follows:
1. In the manufacturing container the following were added:
   Propylene Glycol
   Polyethylene Glycol 400
   Klucel HF
2. Mixed using a Silverson Mixer while heated to about 50°C (45°C -55°C) until a uniform gel was obtained
3. Into the mixture in Step 2 the following were added with mixing until completely dissolved:
   Methyl Salicylate
   Methyl Nicotinate
4. Cooled the mixture to room temperature
5. Added α-Tocopherol (Vitamin E alcohol) and mixed until dissolved.

### Example 10.

| Ingredient | (% w/w) |
|---|---|
| Methyl Salicylate | 0.20 |
| Menthyl Lactate | 0.20 |
| Polyethylene Glycol 400 | 75.00 |
| Propylene Glycol | 24.20 |
| Hydroxypropylcellulose (Klucel HF) | 0.30 |
| Dl-A-Tocopherol (Vitamin E Alcohol) | 0.10 |
| | |
| Total | 100.00 |

The above composition of Example 10 was prepared as follows:
1. In the manufacturing container the following were added:
   Propylene Glycol
   Polyethylene Glycol 400
   Klucel HF
2. Mixed using a Silverson Mixer while heated to about 50°C (45°C -55°C) until a uniform gel was obtained
3. Into the mixture in Step 2 the following were added with mixing until completely dissolved
   Methyl Salicylate
   Menthyl Lactate
4. Cooled the mixture to room temperature
5. Added α-Tocopherol (Vitamin E alcohol) and mixed until dissolved.

### Example 11.

### (K-Y Warming Liquid® Formulation with 0.2% Niacin)

The above composition of Example 11 was prepared as follows:
1. In the manufacturing container the following were added:

| Ingredients | % w/w |
|---|---|
| K-Y warming Liquid (distributed by | 99.8 Personal |
| products Company, division | |
| of McNeil-PPC, Inc., Skillman, | |
| NJ 08558-9418) | |
| Niacin | 0.2 |

2. Mixed using a Silverson Mixer while heated to about 50°C(45°C -55°C) until a uniform liquid is obtained
3. Cooled to room temperature

### Example 12.

(Romanta Therapy ®, Distributed by Passion, Las Vegas, NV 89119-4436)

| Ingredients | % w/w (Not Known) |
|---|---|
| Whole leaf Aloe Vera Concentraate | |
| Purified Water | |
| Sorbitol USP | |
| Hydroxyethylcellulose | |
| Saw Palmetto Extract | |
| Soy Protein | |
| Peppermint USP | |
| Complex 5 (A proprietary blend of five essential ingredients) | |
| L-Arginine, USP | |
| Stevia | |
| Methylparaben USP | |

### Example 13.

### (K-Y Warming Ultragel® Formulation with 0.2% Niacin)

The above composition of Example 13 was prepared as follows:
1. In the manufacturing container add the following:

| Ingredients | % w/w |
|---|---|
| K-Y Warming Ultragel (Distributed by Personal Products Company, division of McNeil-PPC, Inc., Skillman, NJ 08558-9418.) | 99.8 |
| Niacin | 0.2 |

2. Mix using a Silverson Mixer while heating to about 50°C(45°C - 55°C) until a uniform gel is obtained
3. Cool to room temperature

### Example 14.

### (K-Y Liquid® Formulation with 0.2% Niacin)

The above composition of Example 14 was prepared as follows:
1. In the manufacturing container the following were added:

| Ingredients | % w/w |
|---|---|
| K-Y Liquid (Distributed by Personal Products Company, division of McNeil-PPC, Inc., Skillman, NJ 08558-9418.) | 99.8 |
| | |
| Niacin | 0.2 |

2. Mixed using a Silverson Mixer while heated to about 50°C (45°C -55°C) until a uniform liquid was obtained
3. Cooled to room temperature

### Example 15.

### (Excite®, Distributed by Jordyn Nicole)

| | % w/w Not Known |
|---|---|
| Demineralized Water | |
| Sodium Benzoate | |
| Potassium Sorbate | |
| Arginine | |
| Lysine | |
| Horny Goat weed Extract | |
| Methylparaben | |
| Glycerin | |
| Sorbitol | |
| Hydroxymethylcellulose | |
| Vitamin E | |

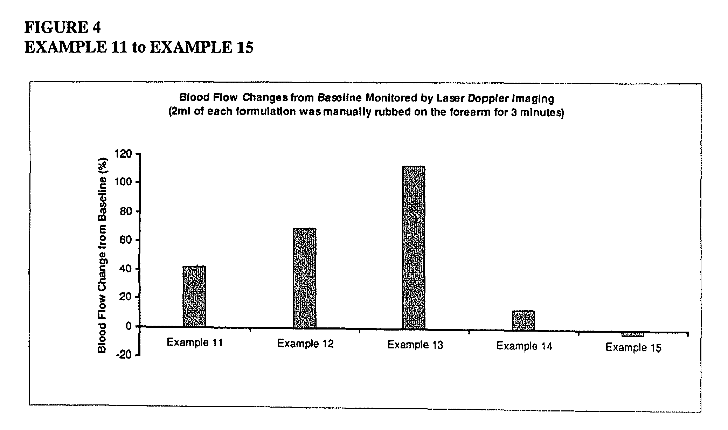

A bar graph of the blood flow changes from baseline monitored by LDI after 2 ml of the compositions of Examples 11-15 were manually rubbed of the forearm of subjects is set forth in Figure 4. Figure 4 demonstrates the following:
(a)Anhydrous Example 13, (KY Warming Ultragel with 0.2% niacin) is superior to aqueous Example 14 (KY Liquid 0.2% niacin). It is also superior to Example 12 (Romanta® Therapy) and Example 15 (Excite®);
(b)Anhydrous Example 13, (KY Warming Ultragel with 0.2% niacin) is also superior to Anhydrous Example 11(K-Y Warming Liquid with 0.2% Niacin; and
(c)Anhydrous Example 13, is superior to Example 11, since it contains 75% Polyethylene glycol as compared to 25% Polyethylene Glycol 400 for Example 11.

### Example 16. (Placebo Anhydrous)

| Ingredient | % w/w |
|---|---|
| Glycerin | 25.00 |
| Propylene Glycol | 75.00 |
| Total | 100.00 |

The above composition of Example 16 was made as follows:
1. Into the manufacturing container the following were added:
   Glycerin
   Propylene Glycol
2. Mixed using a Silverson Mixer until a uniform solution was obtained.

### Example 17. (Anhydrous with 0.2% Niacin and 0.3% Niacinamide)

| Ingredient | % w/w |
|---|---|
| Glycerin | 25.00 |
| Propylene Glycol | 74.50 |
| Niacin | 0.20 |
| Niacinamide | 0.30 |
| Total | 100.00 |

The above composition 17 was made as follows:
1. Into the manufacturing container the following were added:
   Glycerin
   Propylene Glycol
   Niacin
   Niacinamide
2. Mixed using a Silverson Mixer while heated to about 50°C (45°C -55°C) until a uniform gel was obtained.
3. Cooled to room temperature

### Example 18. (Aqueous Placebo)

| Ingredient | % w/w |
|---|---|
| Propylene Glycol | 35.00 |
| Purified Water | 65.00 |
| Total | 100.00 |

The above composition 18 was made as follows:
1. Into the manufacturing container add the following were added:
   Propylene Glycol
   Water
2. Mixed using a Silverson Mixer until a uniform solution was obtained.

### Example 19. (Aqueous Compositions containing 0.2% Niacin and 0.3% Niacinamide)

| Ingredient | % w/w |
|---|---|
| Propylene Glycol | 35.00 |
| Niacin | 0.20 |
| Niacinamide | 0.30 |
| Purified Water | 64.50 |
| Total | 100.00 |

The above composition of Example 19 was made as follows:
1. Into the manufacturing container the following were added:
   Propylene Glycol
   Niacin
   Niacinamide
   Water
2. Mixed using a Silverson Mixer until a uniform solution was obtained.

### Example 20. (Aqueous Composition containing 2% Arginine)

| Ingredient | % w/w |
|---|---|
| Propylene Glycol | 35.00 |
| Arginine | 2.00 |
| Purified Water | 63.00 |
| Total | 100.00 |

The above composition of Example 20 was made as follows:
1. Into the manufacturing container the following were added:
   Propylene Glycol
   Arginine
   Purified Water
2. Mixed using a Silverson Mixer until a uniform solution was obtained.

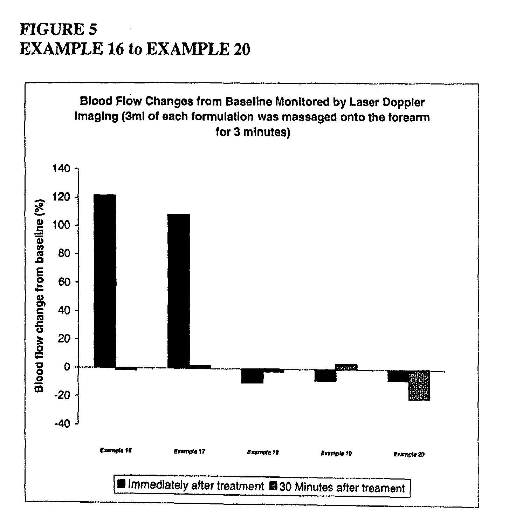

Figure 5 is a bar graph of the blood flow changes from baseline monitored by LDI after 3ml of the compositions of examples 16-20 were manually rubbed onto the forearm of a subject for three minutes in a separate test at a different time for each example.

Figure 5 is an Example of comparison of anhydrous vs. aqueous compositions.

Figure 5 demonstrates the following:
(a) Anhydrous Example 17 containing 2% niacin and 0.3% niacinamide and anhydrous placebo Example 16 are superior in increasing % blood flow as compare with Aqueous Composition Example 19 containing 0.2% niacin and 0.3% niacinamide and the corresponding Aqueous Placebo Example 18 and Example 20 Aqueous Composition containing 2.0% Arginine.
(b) Higher % Blood Flow change for Example 16 (Placebo of Anhydrous Composition for Example 17)), is higher than Example 17, due to higher duration of application as it was applied 3 minutes earlier than example 17 and therefore on the skin of the forearm for three minutes longer than Example 17.

Certain patents describe the use of L-Arginine in compositions to enhance sexual response due to the involvement of L-Arginine in the physiological pathway that leads to vasodilation and, ultimately, engorgement of the sexual organs as L-Arginine is a nitric oxide donor. While L-Arginine is a vasodilator in the tissue where Nitric Oxide Synthetase is present, there is no reported evidence that this nitric oxide-generating enzyme in the human male or female sex organs or tissues necessarily relates to the arousal and/or orgasm processes. Surprisingly, we have found that an L-Arginine containing Aqueous composition (as shown in Figure 5) and the L-Arginine containing product Excite® (as shown in Figure 4), do not induce substantial vasodilation in accordance with the Laser Doppler Imaging test.

### Example 21.

| Ingredient | (% w/w) |
|---|---|
| Polyethylene Glycol 400 | 75.00 |
| Propylene Glycol | 24.40 |
| Hydroxypropylcellulose (Klucel HF) | 0.50 |
| Dl-A-Tocopherol (Vitamin E Alcohol) | 0.10 |
| Total | 100.00 |

The above composition of Example 21 was prepared as follows:
1. In the manufacturing container the following were added:
   Propylene Glycol
   Polyethylene Glycol 400
   Klucel HF
2. Mixed in a Silverson Mixer while heated to about 50°C (45°C -55°C) until a uniform gel was obtained
3. Cooled the mixture to room temperature
4. Added α-Tocopherol (Vitamin E alcohol) and mixed until dissolved.

### Example 22.

| Ingredient | (% w/w) |
|---|---|
| Niacin | 0.10 |
| Optamint | 0.10 |
| Polyethylene Glycol 400 | 75.00 |
| Propylene Glycol | 24.20 |
| Hydroxypropylcellulose (Klucel HF) | 0.50 |
| Dl-A-Tocopherol (Vitamin E Alcohol) | 0.10 |
| Total | 100.00 |

The above composition of Example 22 was prepared as follows:
1. In the manufacturing container the following were added:
   Propylene Glycol
   Polyethylene Glycol 400
   Klucel HF
2. Mixed using a Silverson Mixer while heated to about 50°C (45°C -55°C) until a uniform gel was obtained
3. Into the mixture in Step 2 the following were added with mixing until completely dissolved:
   Niacin
   Optamint
4. Cooled the mixture to room temperature
5. Added α-Tocopherol (Vitamin E alcohol) and mixed until dissolved.

### Example 23.

| Ingredient | (% w/w) |
|---|---|
| Niacin | 0.30 |
| Optamint | 0.10 |
| Polyethylene Glycol 400 | 75.00 |
| Propylene Glycol | 24.00 |
| Hydroxypropylcellulose (Klucel HF) | 0.50 |
| Dl-A-Tocopherol (Vitamin E Alcohol) | 0.10 |
| Total | 100.00 |

The above composition of Example 23 was prepared as follows:
1.In the manufacturing container the following were added:
   Propylene Glycol
   Polyethylene Glycol 400
   Klucel HF
2. Mixed using a Silverson Mixer while heated to about 50°C (45°C -55°C) until a uniform gel was obtained
3. Into the mixture in Step 2 the following were added with mixing until completely dissolved
   Niacin
   Optamint®
4. Cooled the mixture to room temperature
5. Added α-Tocopherol (Vitamin E alcohol) and mixed until dissolved.

### Example 24.

| Ingredient | (% w/w) |
|---|---|
| Niacin | 0.30 |
| Optamint | 0.20 |
| Polyethylene Glycol 400 | 75.00 |
| Propylene Glycol | 23.90 |
| Hydroxypropylcellulose (Klucel HF) | 0.50 |
| Dl-A-Tocopherol (Vitamin E Alcohol) | 0.10 |
| Total | 100.00 |

The above composition of Example 24 was prepared as follows:
1. In the manufacturing container the following were added:
   Propylene Glycol
   Polyethylene Glycol 400
   Klucel HF
2. Mixed using a Silverson Mixer while heated to about 50°C (45°C -55°C) until a uniform gel was obtained
3. Into the mixture in Step 2 the following were added with mixing until completely dissolved
   Niacin
   Optamint
4. Cooled the mixture to room temperature
5. Added α-Tocopherol (Vitamin E alcohol) and mixed until dissolved.

### Example 25.

| Ingredient | (% w/w) |
|---|---|
| Ginseng | 0.10 |
| Optamint | 0.10 |
| Polyethylene Glycol 400 | 75.00 |
| Propylene Glycol | 24.20 |
| Hydroxypropylcellulose (Klucel HF) | 0.50 |
| Dl-A-Tocopherol (Vitamin E Alcohol) | 0.10 |
| Total | 100.00 |

The above composition of Example 25 was prepared as follows:
1. In the manufacturing container add the following:
   Propylene Glycol
   Polyethylene Glycol 400
   Klucel HF
2. Mixed using a Silverson Mixer while heating to about 50°C (45°C -55°C) until a uniform gel was obtained
3. Into the mixture in Step 2 the following were added with mixing until completely dissolved
   Ginseng
   Optamint
4. Cooled the mixture to room temperature
5. Added α-Tocopherol (Vitamin E alcohol) and mixed until dissolved.

### Example 26.

| Ingredient | (% w/w) |
|---|---|
| Benzyl Nicotinate | 0.10 |
| Optamint | 0.10 |
| Polyethylene Glycol 400 | 75.00 |
| Propylene Glycol | 24.20 |
| Hydroxypropylcellulose (Klucel HF) | 0.50 |
| Dl-A-Tocopherol (Vitamin E Alcohol) | 0.10 |
| Total | 100.00 |

The above composition of Example 26 was prepared as follows:
1. In the manufacturing container the following were added:
   Propylene Glycol
   Polyethylene Glycol 400
   Klucel HF
2. Mixed using a Silverson Mixer while heated to about 50°C (45°C -55°C) until a uniform gel was obtained
3. Into the mixture in Step 2 the following were added with mixing until completely dissolved
   Benzyl Nicotinate
   Optamint
4. Cooled to room temperature
5. Added α-Tocopherol (Vitamin E alcohol) and mixed until dissolved.

### Example 27.

| Ingredient | (% w/w) |
|---|---|
| Yohimbine | 0.10 |
| Optamint | 0.10 |
| Polyethylene Glycol 400 | 75.00 |
| Propylene Glycol | 24.20 |
| Hydroxypropylcellulose (Klucel HF) | 0.50 |
| Dl-A-Tocopherol (Vitamin E Alcohol) | 0.10 |
| Total | 100.00 |

The above composition of Example 27 was prepared as follows:
1. In the manufacturing container the following were added:
   Propylene Glycol
   Polyethylene Glycol 400
   Klucel HF
2. Mixed using a Silverson Mixer while heated to about 50°C 45°C -55°C) until a uniform gel was obtained
3. Into the mixture in Step 2 the following were added with mixing until completely dissolved
   Yohimbine
   Optamint
4. Cooled the mixture to room temperature
5. Added α-Tocopherol (Vitamin E alcohol) and mixed until dissolved.

### Example 28. (Vibrel®)

(Vibrel®, manufactured for GlycoBiosciences Inc. Campbellville, Canada

### Ingredient

Purified water
Niacin
Polivinyl Alcohol
Methoxypolyethylene glycol
Glyceron
Propylene Glycol
Carboxymethylcellulose
Hydroxyethylcellulose

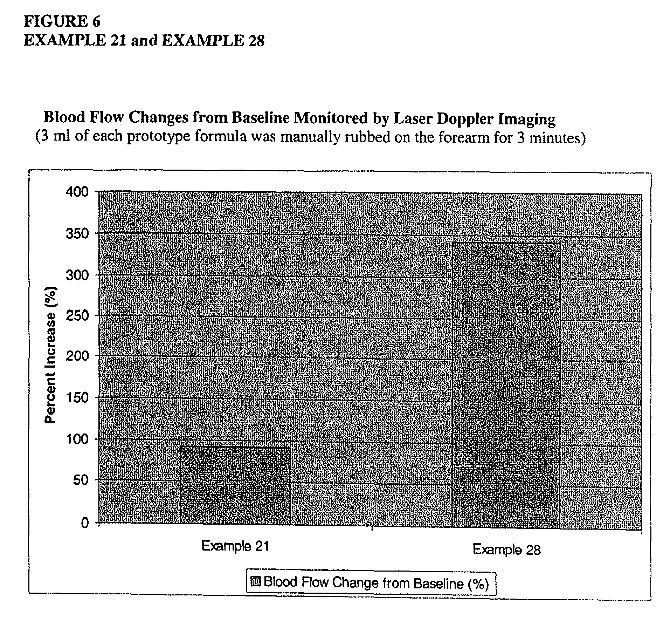

Figure 6 compares following Examples: Example 21 (Placebo of Anhydrous Composition for Example 22) and Example 28 (Vibrel® by www. Vibrel.com). Figure 6 is an Example of "Flushing" product represented by Vibrel (Example 28) as compared with Example 21 (Placebo of Anhydrous Composition for Example 22). Blood Flow change for Vibrel is almost 350% as compared to 90% for Example 21. This exceedingly high blood flow change is responsible for flushing. Also, as demonstrated by Figure 2, Examples 2 and 3 containing 0.1 and 0.5% niacin respectively did not result in an excessive high blood flow change with both well below 350%.

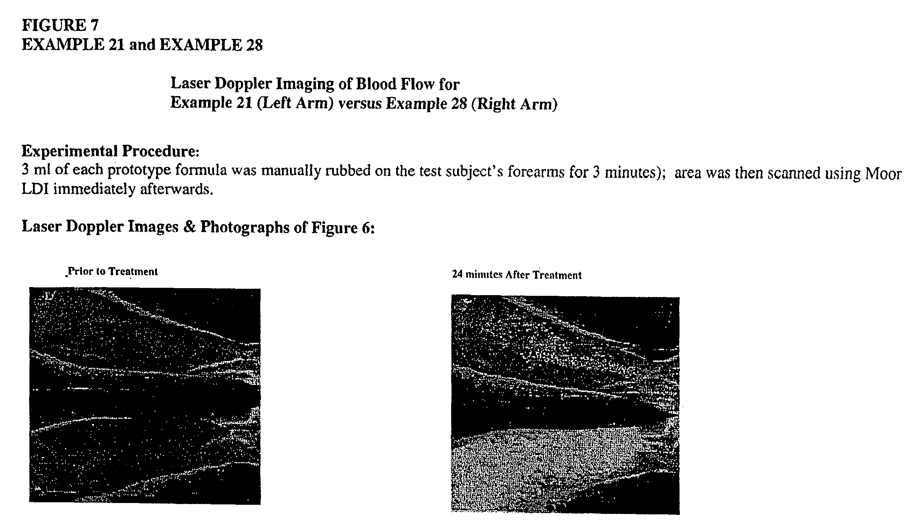

Further, Figure 7 is an LDI image of the skin of the right and left arms after application for 3 minutes of the compositions of Example 21 (left arm) and Example 28 (right arm).

The right arm for Example 28 (VIBREL®) shows extensive area showing excessive blood flow change representing "Flushing" shown by blue color. This represents change on the superficial skin as compared to deeper layers for right arm represented by red color. Extensive red and blue area of superficial blood flow for Example 28 represents "flushing", as demonstrated, the total area covered is much more extensive.

### Example 29. (Zestra ®)

Distributed by the Women's Consumer Product, Division of QualiLife Phrmaceuticals, Inc. Charleston, SC, 29407

| Ingredient | (% w/w) |
|---|---|
| PA-Free Borage Seed Oil | |
| Evening Primrose Oil | |
| Angelica Extract | |
| Coleus Extract | |
| Vitamin C | |
| Vitamin E | |

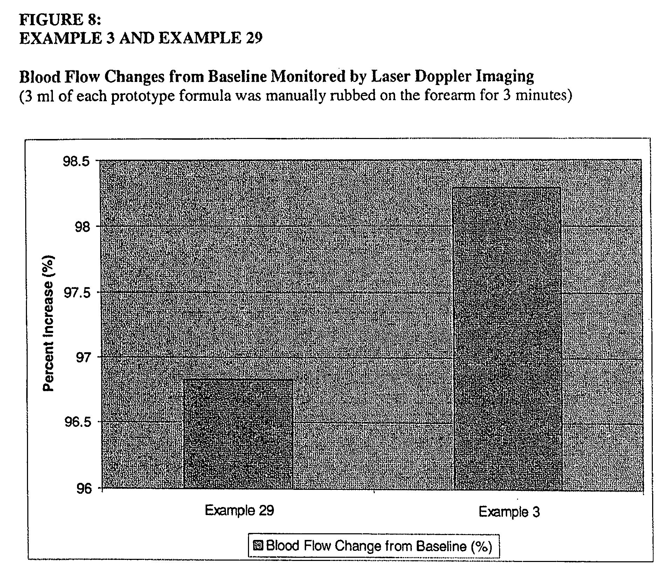

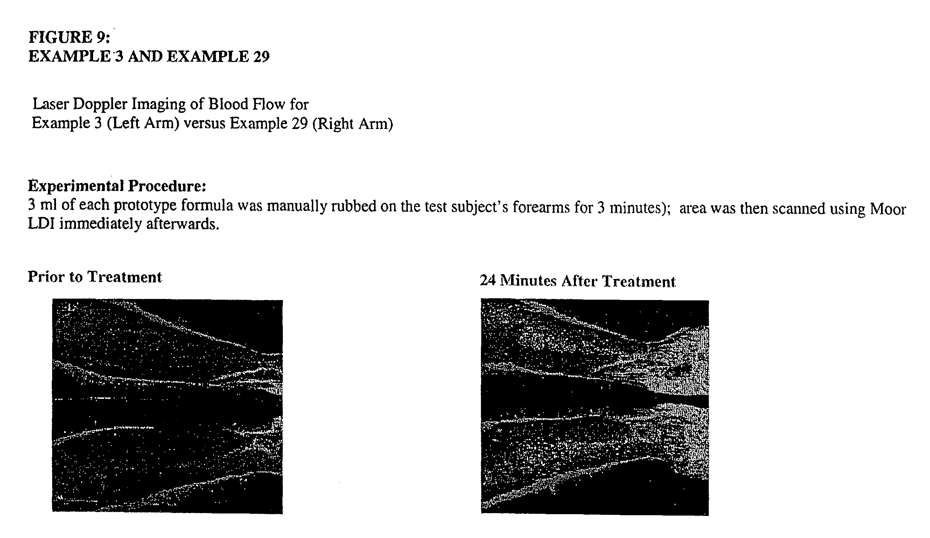

Figure 8 compares Example 3 (Anhydrous composition 0.5% Niacin) and Example 29 (ZESTRA), represented in a bar graph. ZESTRA does not contain any Niacin and, therefore, has a lower blood flow change as demonstrated by Figure 8. Figure 9 is the LDI picture of Figure 8 showing higher % increase in blood flow as represented by greater red and blue area covered for Example 3 as compared with Example 29 (Zestra) showing lower % increase in blood flow as shown by smaller red and blue covered area.

### Example 30A. (Endogenous Vasodilator - Bradykinin - Aqueous Base)

| Ingredient | (% w/w) |
|---|---|
| Purified water | 99.90 |
| Bradykinin | 0.10 |
| | |
| Total | 100.00 |

The above composition of Example 30A was prepared as follows:
1. In the manufacturing container the following were added:
   Purified Water, USP
   Bradykinin
2. Mixed using a Silverson Mixer until a uniform solution was obtained.

### Example 30B. (Endogenous Vasodilator - Bradykinin - Non-aqueous Base)

| Ingredient | (% w/w) |
|---|---|
| Polyethylene Glycol 400 | 75.00 |
| Propylene Glycol | 24.60 |
| Hydroxypropylcellulose (Klucel HF) | 0.30 |
| Dl-A-Tocopherol (Vitamin E Alcohol) | 0.10 |
| Bradykinin | 0.10 |
| Total | 100.00 |

The above composition of Example 30B was prepared as follows:
1. In the manufacturing container the following were added:
   Propylene Glycol
   Polyethylene Glycol 400
   Klucel HF
2. Mixed using a Silverson Mixer while heated to about 50°C (45°C -55°C) until a uniform gel was obtained
3. Into the mixture in Step 2 the following were added with mixing until completely dissolved:
   Bradykinin
4. Cooled to room temperature
5. Added α-Tocopherol (Vitamin E alcohol) and mixed until dissolved.

### Example 31A. (Exogenous Vasodilator/Nitric Oxide Inducer - Isosorbide Dinitrate - Aqueous Base)

| Ingredient | (% w/w) |
|---|---|
| Purified water | 99.90 |
| Isosorbide Dinitrate | 0.10 |
| | |
| Total | 100.00 |

The above composition of Example 31A was prepared as follows:
1. In the manufacturing container the following were added:
   Purified Water, USP
   Isosorbide Dinitrate
2. Mixed using a Silverson Mixer until a uniform solution was obtained.

### Example 31B. (Endogenous Vasodilator - Isosorbide Dinitrate - Non-aqueous Base)

| Ingredient | (% w/w) |
|---|---|
| Polyethylene Glycol 400 | 75.00 |
| Propylene Glycol | 24.60 |
| Hydroxypropylcellulose (Klucel HF) | 0.30 |
| Dl-A-Tocopherol (Vitamin E Alcohol) | 0.10 |
| Isosorbide Dinitrate | 0.10 |
| Total | 100.00 |

The above composition of Example 31B was prepared as follows:
1. In the manufacturing container the following were added:
   Propylene Glycol
   Polyethylene Glycol 400
   Klucel HF
2. Mixed using a Silverson Mixer while heated to about 50°C (45°C -55°C) until a uniform gel was obtained
3. Into the mixture in Step 2 the following were added with mixing until completely dissolved:
   Isosorbide Dinitrate
4. Cooled to room temperature
5. Added α-Tocopherol (Vitamin E alcohol) and mixed until dissolved.

### Example 32A. (Exogenous Vasodilator/PDE5 Inhibitor - Sildenafil citrate - Aqueous Base)

| Ingredient | (% w/w) |
|---|---|
| Purified water | 99.90 |
| Sildenafil citrate | 0.10 |
| | |
| Total | 100.00 |

The above composition of Example 32A was prepared as follows:
1. In the manufacturing container the following were added:
   Purified Water, USP
   Sildenafil citrate
2. Mixed using a Silverson Mixer until a uniform solution was obtained.

### Example 32B. (Endogenous Vasodilator - Sildenafil citrate - Non-aqueous Base)

| Ingredient | (% w/w) |
|---|---|
| Polyethylene Glycol 400 | 75.00 |
| Propylene Glycol | 24.60 |
| Hydroxypropylcellulose (Klucel HF) | 0.30 |
| Dl-A-Tocopherol (Vitamin E Alcohol) | 0.10 |
| Sildenafil citrate | 0.10 |
| Total | 100.00 |

The above composition of Example 32B was prepared as follows:
1. In the manufacturing container the following were added:
   Propylene Glycol
   Polyethylene Glycol 400
   Klucel HF
2. Mixed using a Silverson Mixer while heated to about 50°C (45°C -55°C) until a uniform gel was obtained
3. Into the mixture in Step 2 the following were added with mixing until completely dissolved:
   Bradykinin
4. Cooled to room temperature
5. Added α-Tocopherol (Vitamin E alcohol) and mixed until dissolved.

### Example 33. Blood Flow Changes Using Compositions of Examples 30A, 30B, 31A, 31B, 32A and 32B

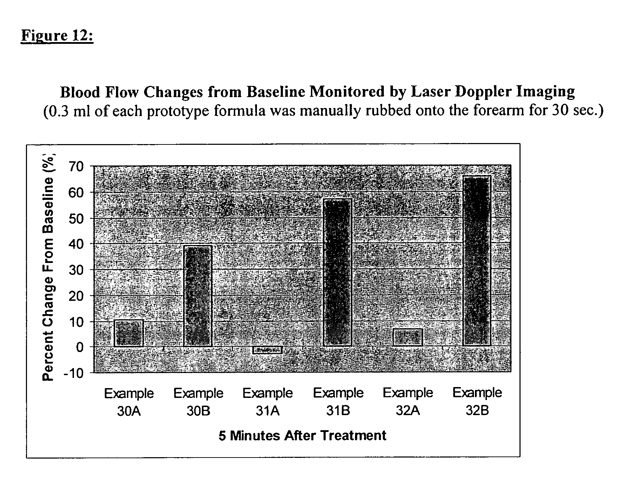

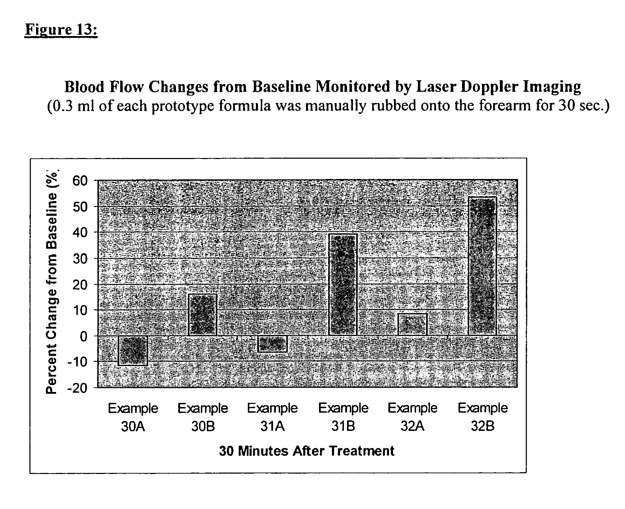

The compositions of Examples 30A, 30B, 31A, 31B, 32A and 32B were applied in the amount of 0.3 ml per application to the forearms of a subject. The blood flow was measured using LDI techniques prior to application, five minutes after application and 30 minutes after application of the compositions of the examples. Redness, irritation, itching of the skin (i.e., "flushing") was not observed to be caused by any of the sample compositions, even in the cases of Examples 30B, 31B and 32B, in which increased blood flow was observed. The difference in the rate of blood flow was calculated on a percentage basis for each of the sample compositions. The differences after five minutes are set forth in the graph of Figure 12. The differences after thirty minutes are set forth in the graph of Figure 13.

Example 34. Comparison of Blood Flow Between Examples 30B, 31B, 32B and Example 22.

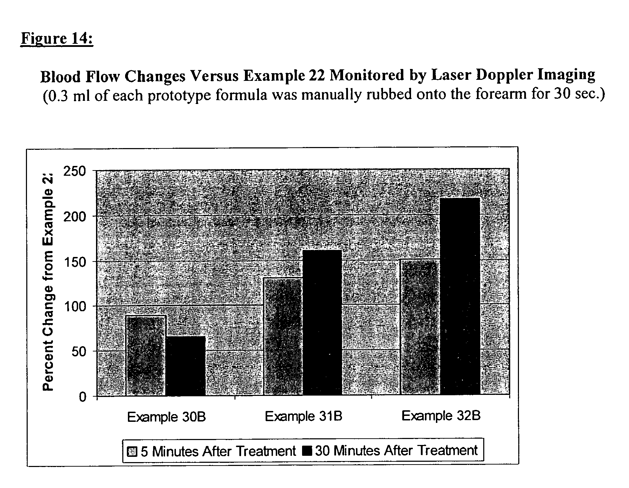

The compositions of Examples 22, 30B, 31B, and 32B were applied in the amount of 0.3 ml per application to the forearms of a subject. The blood flow was measured using LDI techniques prior to application, five minutes after application and 30 minutes after application of the compositions of the examples. Redness, irritation, itching of the skin (i.e., "flushing") was not observed to be caused by any of the sample compositions, even in the cases of Examples 30B, 31B and 32B, in which increased blood flow was observed. The difference in the rate of blood flow was calculated on a percentage basis for the Examples 30B, 31B and 32B sample compositions in relation to percentage increase over Example 22. The differences after five minutes and thirty minutes are set forth in the graph of Figure 14.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

## Claims

1. An anhydrous composition comprising a vasodilator and an acceptable carrier wherein said vasodilator is present in an amount effective to increase the blood flow to human tissue when said composition is applied to said human tissue.

2. A composition according to claim 1, wherein said vasodilator is capable of penetration to the deeper layers of said tissue and does not cause flushing on said tissue.

3. A composition according to claim 1, wherein said vasodilator is present in an amount of from about 0.1 to about 0.5% by weight.

4. A composition according to claim 1, wherein said composition is free of a niacin derivative.

5. A composition according to claim 1, wherein said carrier is a polyhydric alcohol.

6. A composition according to claim 5, wherein said polyhydric alcohol is selected from polyethylene glycol, polyethylene glycol ethers, propylene glycol, hexalene glycol, butylene glycol and mixtures thereof.

7. A composition according to claim 1, wherein said human tissue is the genital region of a male or of a female.

8. A composition according to claim 6 wherein said polyhydric alcohol is a mixture of polyethylene glycol and propylene glycol.

9. A composition according to claim 8, wherein the weight ratio of said polyethylene glycol to said propylene glycol is about 3:1.

10. A composition according to claim 1, further comprising an antioxidant in an amount effective to prevent the degradation of said polyhydric alcohols.

11. A composition according to claim 10, wherein said antioxidant is selected from the group consisting of tocopherol, ascorbic acid and butylated hydroxytoluene (BHT), wherein said polyhydric alcohol is selected from the group consisting of propylene glycol, polyethylene glycol, polyethylene glycol ethers, butylethylene glycol, hexalene glycol and combinations thereof.

12. A composition according to claim 10, wherein said antioxidant is present in an amount of from about 0.05% to about 3%

13. A composition according to claim 12, wherein said antioxidant is selected from α-tochopherol, α-tochopherol acetate, and mixtures thereof.

14. A composition according to claim 1, further comprising an effective amount of at least one sensitivity enhancer.

15. A composition according to claim 14, wherein said sensitivity enhancer is present in an amount ranging from about 0.05 to about 5% by weight.

16. A composition according to claim 15, wherein said sensitivity enhancer is selected from a cooling compound, a warming compound, a tingling compound, and mixtures thereof.

17. A composition according to claim 16, wherein said cooling compound is selected from 2-Isopropyl-N, 2,3-trimethylbutyramide, N-Ethyl-p-menthane-3-carboxamide and Ethyl 3-(p-menthane-3-carboxamido) acetate Menthone glycerol ketal, (-)-Menthyl lactate, (-)-Isopulegol, Alpha Glucosyl Hisperidin and mixtures thereof.

18. A composition according to claim 15, wherein said warming compound is selected from piperine, 1- Acetoxychavicol Acetate, α - hydroxyshansool, Timurol, Hesperidin, ginger extract, and mixtures thereof.

19. A composition according to claim 14, wherein said tingling compound is selected from Shansools, Spilanthol, Timurol and mixtures thereof.

20. A composition according to claim 1, further comprising an effective amount of a lubricating and viscosity agent.

21. A composition according to claim 20, wherein said lubricating agent is selected from the group consisting of carboxymethylcellulose, hydroxyethylcellulose, hydroxypropyl methylcellulose, hydroxypropylcellulose, and mixtures thereof.

22. A composition according to claim 21, wherein said lubricating agent is present in an amount of from about 0.05 to about 5% by weight.

23. A composition according to claim 1, wherein said composition is non-flushing and wherein said increase in blood flow is less than a 300% increase.

24. A composition according to claim 1, wherein said composition is capable of penetrating the epidermis of said human tissue when applied to said human tissue.

25. A method of attaining enhanced sexual wellness of an individual comprising administering to the genital areas of said individual, an anhydrous composition comprising an effective amount of a vasodilator and an acceptable carrier.

26. A method of claim 25, wherein said composition is administered between 5 minutes to 30 minutes prior to intercourse.

27. A method according to claim 25, wherein said vasodilator is present in an amount of from about 0.1 to about 0.5% by weight.

28. A method according to claim 25, wherein said composition is capable of penetrating the epidermis of said human tissue when applied to said human tissue.

29. A method according to claim 25, wherein said carrier is a polyhydric alcohol.

30. A method according to claim 29, wherein said polyhydric alcohol is selected from polyethylene glycol, propylene glycol, polyethylene glycol ethers, hexalene glycol, butylenes glycol and mixtures thereof.

31. A method according to claim 29, wherein said polyhydric alcohol is a mixture of polyethylene glycol and propylene glycol.

32. A method according to claim 31, wherein said weight ratio of said polyethylene glycol to said propylene glycol is about 3:1.

33. A method according to claim 25, wherein said composition comprises an antioxidant in an amount effective to prevent the degradation of said polyhydric alcohols.

34. A method according to claim 33, wherein said antioxidant is selected from the group consisting of tocopherol, ascorbic acid and butylated hydroxytoluene (BHT), wherein said polyhydric alcohol is selected from the group consisting of propylene glycol, polyethylene glycol, butylethylene glycol, hexalene glycol and combinations thereof.

35. A method according to claim 33, wherein said antioxidant is present in an amount of from about 0.05% to about 3%

36. A method according to claim 35, wherein said antioxidant is selected from α-tochopherol, α-tochopherol acetate, and mixtures thereof.

37. A method according to claim 25, wherein said composition comprises an effective amount of at least one sensitivity enhancer.

38. A method according to claim 37, wherein said sensitivity enhancer is present in an amount ranging from about 0.05 to about 5% by weight.

39. A method according to claim 37, wherein said sensitivity enhancer is selected from a cooling compound, a warming compound, a tingling compound, and mixtures thereof.

40. A method according to claim 39, wherein said cooling compound is selected from 2-Isopropyl-N, 2,3-trimethylbutyramide, N-Ethyl-p-menthane-3-carboxamide and Ethyl 3-(p-menthane-3-carboxamido) acetate Menthone glycerol ketal, (-)-Menthyl lactate, (-)-Isopulegol, Alpha Glucosyl Hisperidin and mixtures thereof.

41. A method according to claim 39, wherein said warming compound is selected from piperine, 1- Acetoxychavicol Acetate, α - hydroxyshansool, Timurol, Hesperidin, ginger extract, and mixtures thereof.

42. A method according to claim 39, wherein said tingling compound is selected from Shansools, Spilanthol, Timurol and mixtures thereof.

43. A method according to claim 25, wherein said composition comprises an effective amount of a lubricating and viscosity agent.

44. A method according to claim 44, wherein said lubricating agent is selected from the group consisting of carboxymethylcellulose, hydroxyethylcellulose, hydroxypropyl methylcellulose, hydroxypropylcellulose, and mixtures thereof.

45. A method according to claim 44, wherein said lubricating agent is present in an amount of from about 0.05 to about 5% by weight.

46. A method according to claim 25, wherein said composition is non-flushing and wherein said increase in blood flow is less than a 300% increase.

47. A composition according to claim 1 wherein said vasodilator is selected from the group consisting of endogenous and exogenous vasodilators.

48. A composition according to claim 48 wherein said vasodilator comprises an endogenous vasodilator selected from the group consisting of nitric oxide, histamine, prostaglandin D2, adenosine, L-arginine and bradykinin.

49. A composition claim 48 wherein said vasodilator comprises an exogenous vasodilator selected from the group consisting of nitric oxide inducers and PDE5 inhibitors.

50. A composition according to claim 50 wherein said exogenous vasodilator comprises a nitric oxide inducer selected from the group consisting of glyceryl nitrate (commonly known as nitroglycerin), isosorbide mononitrate, isosorbide dinitrate, pentaerythrotol tetranitrate and sodium nitroprusside.

51. A composition according to claim 50 wherein said exogenous vasodilator comprises a PDE5 inhibitor selected from the group consisting of sildenafil, tadalafil, vardenafil, theobromine and papaverine.

52. A composition according to claim 1 wherein said vasodilator is selected from the group consisting of niacin and niacin derivatives.
